(19) Europäisches Patentamt
European Patent Office
Office européen des brevets

(11) Veröffentlichungsnummer: **0 220 220 B1**

# (12) EUROPÄISCHE PATENTSCHRIFT

(45) Veröffentlichungstag der Patentschrift: **23.05.90**

(51) Int. Cl.$^5$: **C 07 C 255/10,** C 07 C 255/17, C 07 C 255/35, C 07 C 255/40 // A01N37/42

(21) Anmeldenummer: **86902405.9**

(22) Anmeldetag: **16.04.86**

(86) Internationale Anmeldenummer: **PCT/EP86/00220**

(87) Internationale Veröffentlichungsnummer: **WO 86/06369 06.11.86 Gazette 86/24**

## (54) Verfahren zur Herstellung von 2,2,-Dibrom-3-oxonitrilen.

(30) Priorität: **24.04.85 DE 3514688**

(43) Veröffentlichungstag der Anmeldung: **06.05.87 Patentblatt 87/19**

(45) Bekanntmachung des Hinweises auf die Patenterteilung: **23.05.90 Patentblatt 90/21**

(84) Benannte Vertragsstaaten: **AT BE CH DE FR GB IT LI NL SE**

(56) Entgegenhaltungen:
**EP-A-0 089 011**
**FR-A-2 239 456**

**Chemical Abstracts, vol. 61, No. 12, 7 December 1964, Columbus, Ohio, US. H.Gault et al.: "Condensation of ethyl diethoxyacetate with acetonitrile 4,4-diethoxy-3-oxobutyronitrile", see column 14523c**

(73) Patentinhaber: **Henkel Kommanditgesellschaft auf Aktien**
**Postfach 1100 Henkelstrasse 67**
**D-4000 Düsseldorf-Holthausen (DE)**

(72) Erfinder: **KRAUSE, Horst-Jürgen**
**Am Nettchesfeld 22**
**D-4000 Düsseldorf 13 (DE)**
Erfinder: **LEINEN, Hans-Theo**
**Gertrudisstrasse 2**
**D-4000 Düsseldorf (DE)**
Erfinder: **LEHMANN, Rudolf**
**Schnugsheide 2**
**D-5653 Leichlingen (DE)**

(56) Entgegenhaltungen:
**Chemical Abstracts. vol. 83, No. 13, 29 September 1975, Columbus, Ohio, US J. Mikolajczyk et al.: " Synthesis of organic phosphates with insecticidal properties. I. Synthesis of 0,0-dialkyl-0-10(mono-or dichlorophenyl)-2-halo-2-cyanovinyl phosphates", see page 504, abstract 113818e**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 220 220 B1

(56) Entgegenhaltungen:
**Methoden der Organischen Chemie, (Houben-Weyl) 4th edition, published in 1972, Georg Thieme Verlag, Stuttgart (DE), see pages 573-574)**

**Chemical Abstracts, vol. 84, No. 23, 7 June 1976, Columbus, Ohio, US., see page 432, abstract 164406x**

**Beschreibung**

Die Erfindung betrifft ein Verfahren zur Herstellung von 2,2-Dibrom-3-oxonitrilen der Formel I,

$$R—CO—CBr_2—CN \qquad (I)$$

in der R einen geradkettigen oder verzweigten Alkylrest mit 3 bis 21 Kohlenstoffatomen, einen gegebenenfalls mit Alkylresten substituierten cycloaliphatischen Rest mit 3 bis 6 Ringkohlenwasserstoffatomen, oder einen gegebenenfalls mit Alkylresten, Alkoxyresten, Fluor- oder Bromatomen oder Nitrogruppen substituierten Phenyl- oder Naphtylrest darstellt, bei dem man einen Carbonsäureester der Formel II,

$$R—CO—OR' \qquad (II)$$

in der R die für die Formel angegebene Bedeutung hat und in der R' einen geradkettigen oder verzweigten Alkylrest mit 1 bis 4 Kohlenstoffatomen darstellt, in Gegenwart einer starken Base umsetzt, und das entstendende 3-Oxonitril mit Brom umsetzt. Das neue Verfahren ist dadurch gekennzeichnet, daß man den Carbonsäureester der Formel II in Gegenwart von Natriummethylat mit im Überschuß vorhandenen Acetonitril umsetzt und den bei der Reaktion entstehenden Alkohol zusammen mit Acetonitril laufend aus dem Reaktionsgemisch abdestilliert, das entstendene 3-Oxonitril aus dem Reaktionsgemisch isoliert und in einem gegen Brom indifferenten Lösungsmittel und in Gegenwart eines Bromwasserstoffacceptors, gegebenenfalls unter Kühlung mit Brom umsetzt und das entstendene 2,2-Dibromoxonitril der Formel I aus dem Reaktorgemisch isoliert.

Aus Chemical Abstracts, Referat 84:164406x (1976) sind Verbindungen der Formel I bekannt, in der R einen mit 1 bis 3 Chloratomen substituierten Phenylrest bedeutet; diese Literaturstelle enthält keinen Hinweis auf die antimikrobielle Wirksamkeit dieser Substanzklasse. Chemical Abstracts, Bd. 61, Spalte 14523e beschreibt die Herstellung von 4,4-Diethoxy-3-oxo-2,2-dibrombutyronitril aus dem Acetal Ethyl-2,2-diethoxyacetat. Aus der FR—A—2 239 456 ist bekannt, daß aus α-Halogenketeonen in Form ihrer Alkali- oder Erdalkalisalze durch Umsetzung mit der stöchiometrisch erforderlichen Menge Halogen die entsprechenden α,α-Dihalogenketone erhalten werden können.

Die Verwendung der 2,2-Dibrom-3-oxonitrile der Formel I als antimikrobielle Wirkstoffe wird hier nicht beansprucht, sie ist vielmehr Gegenstand der zum gleichen Zeitpunkt hinterlegten Euro-PCT-Patentanmeldung 86902817.5-PCT/EP 8600221 der Patentinhaberin.

Als Ausgangsmaterial für die Herstellung der 2,2-Dibrom-3-oxonitrile der Formel I nach dem oben angegebenen Verfahren eignen sich beispielsweise Ester der folgenden Carbonsäuren: Buttersäure, Valeriansäure, Capronsäure, Caprylsäure, Caprinsäure, Laurinsäure, Myristinsäure, Palmitinsäure, Stearinsäure, Arachidinsäure, Behensäure, Lignocerinsäure, Pivalinsäure, 2,2-Dimethylbuttersäure, 2,2-Dimethylvalerinsäure, 2-Ethylhexancarbonsäure, Cyclopropancarbonsäure, 1-Methylcyclopropancarbonsäure, 2-Ethylcyclopropancarbonsäure, Cyclobutancarbonsäure, 2-Methylcyclopropancarbonsäure, 3-Propylcyclobutancarbonsäure, Cyclopentancarbonsäure, 1-Ethylcyclopentancarbonsäure, 2-Methylcyclopentancarbonsäure, 3-Propylcyclopentancarbonsäure, Cyclohexancarbonsäure, 1-Methylcyclohexylcarbonsäure, 2-Methylcyclohexancarbonsäure, 3,4-Dimethylcyclohexancarbonsäure, Benzoesäure, 2-Methylbenzoesäure, 3-Methylbenzoesäure, 4-Methylbenzoesäure, 2,4-Dimethylbenzoesäure, 2-Fluorbenzoesäure, 3-Fluorbenzoesäure, 4-Fluorbenzoesäure, 2-Brombenzoesäure, 3-Brombenzoesäure, 4-Brombenzoesäure, 2,4-Dibrombenzoesäure, 2,5-Dibrombenzoesäure, 2-Methoxybenzoesäure, 2-Ethoxybenzoesäure, 3-Methoxybenzoesäure, 4-Methoxybenzoesäure, 3,4-Dimethoxybenzoesäure, 2-Nitrobenzoesäure, 3-Nitrobenzoesäure, 4-Nitrobenzoesäure, 2,4-Dinitrobenzoesäure, 2,5-Dinitrobenzoesäure, 3,5-Dinitrobenzoesäure, 2-Trifluormethylbenzoesäure, 4-Trifluormethylbenzoesäure, 1-Naphthalincarbonsäure und 2-Naphthalincarbonsäure.

Die Alkoholkomponente der Carbonsäureester der Formel II kann aus Methanol, Ethanol, n-Propanol, i-Propanol, n-Butanol, i-Butanol, sek.-Butanol und tert-Butanol bestehen, wobei Methylester besonders bevorzugt sind.

Bei der ersten Stufe des oben beschrieben Verfahrens handelt es sich um die aus der organischen Synthese bekannte Kondensation von Carbonsäureestern mit Carbonsäurenitrilen, bei der in Gegenwart starker Basen die entsprechenden 3-Oxonitrile erhalten werden können. Als starke Basen kommen nach den Angaben der einschlägigen Literatur insbesondere feinverteiltes Natriumamid und Natriumhydrid in Betracht (siehe Houben-Weyl: Methoden der organischen Chemie, Band 8, S. 574—575; DE—A—32 09 472 und die entsprechende EP—A—89 011).

Im Gegensatz zu den Angaben in Houben-Weyl konnte in Zusammenhang mit dieser Erfindung gezeigt werden, daß die Kondensation von Fettsäureestern mit Acetonitril auch mit Natriummethylat als Base durchgeführt werden kann, wenn man mit einem größeren Überschuß an Acetonitril arbeitet und den bei der Kondensationsreaktion entstehenden Alkohol zusammen mit Acetonitril laufend aus dem Reaktionsgemisch abdestilliert.

Wird Natriummethylat als Kondensationsmittel verwendet, so setzt man mit Vorteil 10 bis 20 Mol Acetonitril pro Mol Carbonsäureester ein.

Das als Kondensationsmittel vorgesehene Natriummethylat wird zweckmäßigerweise in Mengen von 1 bis 2 Mol pro Mol des vorhandenen Carbonsäureesters eingesetzt.

Im erfindungsgemäßen Verfahren wird im allgemeinen kein Lösungsmittel zugesetzt, wenn man von dem mit dem Natriummethylat normalerweise eingebrachten Methanol absieht.

Es hat sich als zweckmäßig erwiesen, die Kondensationsreaktion in einer Inertgasatmosphäre, beispielsweise in einer Stickstoff- oder Argonatmosphäre durchzuführen. Die Reaktionstemperatur kann im Bereich zwischen 50°, vorzugsweise 60°C und der Rückflußtemperatur des jeweiligen Reaktionsgemisches liegen.

Bei der Durchführung der Kondensationsreaktion legt man ein Gemisch aus Carbonsäureester und Acetonitril vor, erwärmt bis zum Sieden und trägt dann langsam die Natriummethylatlösung ein. Dabei wird der bei der Reaktion einstehende Alkohol und das mit dem Natriummethylat eingebrachte Methanol über eine entsprechende Destillationsvorrichtung zusammen mit Acetonitril laufend aus dem Reaktionsgemisch abgezogen.

Die Aufarbeitung des Reaktionsgemisches erfolgt normalerweise durch Zugabe einer genügend großen Menge Wasser, so daß sich die im Reaktinsgemisch vorhanden Feststoffe unter Bildung eines Zweiphasensystems vollkommen auflösen. Die wäßrige Phase wird abgetrennt, die organische Phase gegebenenfalls mit weiterem Wasser ausgewaschen, die abgetrennten wäßrigen Phasen werden vereinigt, abgekühlt und unter Kühlung mit wäßriger Mineralsäure auf einen pH-Wert zwischen 1 und 5 eingestellt. Dabei scheidet sich das 3-Oxonitril als Festkörper oder als Öl ab und kann in bekannter Weise isoliert werden. Bei der Aufarbeitung des Reaktionsgemisches kann aber auch so vorgegangen werden, daß man den vorhandenen Niederschlag durch Saugfiltration abtrennt, mit einem geeigneten Lösungsmittel wäscht und ihn dann zur Neutralisation unter sehr starkem Rühren in eine wäßrige Mineralsäurelösung einträgt. Dabei wird die Säuremenge so gewählt, daß am Ende der Reaktion die wäßrige Lösung einen pH-Wert zwischen 1 und 5 aufweist. Als Mineralsäure wird Salzsäure bevorzugt.

Zweckmäßigerweise sollte bei der Aufarbeitung des Reaktionsgemisches die Behandlung mit der wäßrigen Mineralsäure bei einer Temperatur durchgeführt werden, die 10°C, vorzugsweise 0°C, nicht übersteigt.

Die in der zweiten Stufe des oben angegebenen Verfahrens zur Herstellung der Verbindungen der Formel I durchgeführte Bromierung der erhaltenen 3-Oxonitrile erfolgt mit elementarem Brom in einem gegen Brom indifferenten Lösungsmittel in Gegenwart eines Bromwasserstoffacceptors.

Als Lösungsmittel eignen sich chlorierte Kohlenwasserstoffe, insbesondere Methylenchlorid, oder Eisessig. Die Arbeitsweise mit Methylenchlorid hat saich besonders bewährt, da in diesem Fall in einem Zweiphasengemisch Lösungsmittel/Wasser gearbeitet werden kann und der Bromwasserstoffacceptor in gelöster Form in der wäßrigen Phase vorliegt.

Brom wird bevorzugt in äquimolaren Mengen, allenfalls in einem Überschuß von 5 bis 10 Mol-%, eingesetzt. Als Bromwasserstoffacceptoren eignen sich vor allemn schwache Basen, wie Natriumhydrogencarbonat, Kaliumhydrogencarbonat, Calciumcarbonat oder Natriumacetat.

Die Bromierung der 3-Oxonitrile wird zweckmäßigerweise unter Kühlung durchgeführt, wobei Temperaturen zwischen −10 bis +25°C bevorzugt sind.

Bei der Durchführung der Bromierung wird das umzusetzende 3-Oxonitril, gelöst in dem jeweiligen Lösungsmittel, und der Bromwasserstoffacceptor, gegebenenfalls in Wasser gelöst, vorgelegt und unter starkem Rühren und gegebenenfalls Kühlen die entsprechende Menge Brom, zweckmäßigerweise ebenfalls in dem gewählten Lösungsmittel gelöst, langsam zugegeben. Nach dem Ende der Bromzugabe wird das Reaktionsgemisch zweckmäßigerweise zur Vervollständigung der Reaktion noch 3 bis 4 Stunden lang bei Raumtemperatur weitergerührt.

Zur Aufarbeitung des Reaktionsgemisches wird zunächst die gegebenenfalls vorhandene wäßrige Phase abgetrennt; die organische Phase wird mit Wasser ausgewaschen. Danach noch vorhandene geringe Mengen Brom können mit Hilfe von Natriumhydrogensulfit oder Hydroxylaminhydrochlorid entfernt werden. Die organische Lösung wird dann mit Hilfe eines geeigneten Trocknungsmittels, beispielsweise wasserfreiem Natriumsulfat, getrocknet und vom Lösungsmittel befreit. Die zurückbleibenden 2,2-Dibrom-3-oxonitrile besitzen in den meisten Fällen bereits einen Reinheitsgrad, der es erlaubt, die Produkte direkt der praktischen Verwendung zuzuführen. Gewünschtenfalls können die Produkt durch Umkristallisieren oder Destillation im Dünnschichtverdampfer gereinigt werden.

Aufgrund ihrer mikrobistatischen und mikrobiziden Wirkung gegenüber Bakterien und Pilzen eigenen sich die erfindungsgemaß hergestellten Substanzen für die Lösung der verschiedenen Desinfektions- und Konservierungsaufgaben im nicht-therapeutischen Bereich. Zur Verwendung als Wirkstoff in antimikrobiellen Mitteln können die erfindungsgemaß hergestellten Substanzen in flüssige, pastenförmige oder feste Zubereitungen eingearbeitet werden. Derartige Mittel können auf den verschiedensten Gebieten zum Einsatz gelangen, beispielsweise als Reinigungs-, Desinfektions- und Konservierungsmittel für Textilien, Fußböden, Krankenhauseinrichtungen, medizinische Instrumente, Schulen, Badeanstalten, öffentliche Verkehrsmittel, gewerbliche Betriebe, wie Molkereien, Brauereien und Wäschereien.

## Beispiele

### A. Herstellung der 2,2-Dibrom-3-oxonitrile

#### Beispiel 1

In einer Rührapparatur mit Rührer, Tropftrichter, Gaseinleitungsrohr und aufgesetzter Destillationskolonne wurden unter Rühren und Einleiten von Stickstoff 130,2 g (1 Mol) Capronsäuremethylester und 548 g (13,4 Mol) Acetonitril zum Sieden erhitzt. In die siedende Reaktionslösung wurden im Verlauf von 2 Stunden 180 g 30-gewichtsprozentige Natriummethylatlösung (54 g = 1 Mol Natriummethylat) eingetropft. Gleichzeitig wurde aus der Natriummethylatlösung stammendes und bei der Reaktion entstehendes Methanol zusammen mit Acetonitril über die Destillationskolonne abdestilliert. Nach dem Ende der Zugabe der Natriummethyllatlösung wurde die Destillation so lange weitergeführt, bis sich im Kolonnenkopf der Siedepunkt des reinen Acetonitrils einstellte und die Methanolabspaltung beendet war. Bei der Destillation wurden insgesamt 640 g Destillat erhalten, das nach gaschromatischer Untersuchung zu 24,1 Gewichtsprozent aus Methanol und zu 75,9 Gewichtsprozent aus Acetonitril bestand.

Das beim Abkühlen des Reaktionsgemisches ausfallende Natriumenolatsalz des 3-Oxooctannitrils wurde durch Filtration abgetrennt und mit Aceton gewaschen. Dabei wurden 134 g (83% d. Th.) weißes Salz erhalten.

Zur Überführung in das freie 3-Oxooctannitril wurden 27,6 g (0,17 Mol) des Natriumenolatsalzes in eine Mischung aus 100 ml Eiswasser und 58 ml 10-gewichtsprozentiger Salzsäure eingerührt und bis pH 4 angesäuert. Das sich abscheidende 3-Oxooctannitril wurde in Methyl-tert.-butylether aufgenommen, die abgetrennte organische Phase wurde mit Wasser neutral gewaschen und über Natriumsulfat getrocknet. Nach dem Abdestillieren des Methyl-tert.-butylethers verbleiben 222,5 g (93% d. Th.) 3-Oxooctannitril, das nach gaschromatographischer Analyse einen Reinheitsgrad von 98 Gewichtsprozent besaß.

In einer Rührapparatur wurden unter Rühren, Eiskühlung und Einleiten von Stickstoff zu 13,9 g (0,1 Mol) 3-Oxooctannitril, gelöst in 75 ml Methylenchlorid, und 22 g (0,22 Mol) Kaliumhydrogencarbonat, gelöst in 75 ml Wasser bei 10 bis 15°C im Verlauf von 90 Minuten 35,2 g (0,22 Mol) Brom, gelöst in 40 ml Methylenchlorid, zugetropft. Die entstehende Mischung wurde zur Vervollständigung der Reaktion 4 Stunden lang bei Raumtemperatur weitergerührt. Anschließend wurde die Methylenchloridphase abgetrennt. Überschüssiges Brom wurde mit etwas Natriumhydrogensulfit entfernt. Danach wurde die Lösung mit Wasser neutral gewaschen und über Natriumsulfat getrocknet. Das nach dem Abdestillieren des Methylenchlorids verbleibende Rohprodukt wurde über einen Dünnschichtverdampfer bei 95°C/0,01 mbar destilliert. Es wurden dabei 22,1 g (75% d. Th.) 2,2-Dibrom-3-oxooctannitril (Substanz A) als gelbliches Öl mit $n_D^{20}$ = 1,5055 erhalten, das nach gaschronomatographischer Analyse einen Reinheitsgrad von 97,2 Gewichtsprozent besaß.

Analyse
$C_8H_{11}NOBr_2$ (MM 296,989)

berechnet (%) : 32,35 C; 3,73 H; 4,72 N; 53,81 Br.
gefunden (%) : 31,9 C; 3,2 H; 5,0 N; 53,5 Br.

$^1$H—NMR-Spektrum (60 MHz; $CDCl_3$)
= 3,10 (t); 1,75 (m); 1,35 (m); 0,90 (t)

In analoger Weise wurden die Substanzen B bis E hergestellt.

Substanz B: 2,2-Dibrom-3-oxodecannitril
gelbliches Öl mit $n_D^{20}$ = 1,4978; Sdp. 120°C/0,01 mbar
Analyse
$C_{10}H_{15}NOBr_2$ (MM 325,042)

berechnet (%) : 36,95 C; 4,65 H; 4,31 Br; 49,17 Br.
gefunden (%) : 37,5 C; 4,65 H; 4,45 Br; 49,3 Br.

$^1$H—NMR-Spektrum (90 MHz; $CDCl_3$)
= 3,01 (t); 1,72 (m); 1,30 (m); 0,87 (t)
$^{13}$C—NMR-Spektrum (50 $MH_2$; $CDCl_3$)
= 189,48 (s); 113,53 (s); 34,02 (t); 33,87 (s); 31,53 (t); 28,80 (t); 28,62 (t); 24,64 (t); 22,53 (t); 14,01 (q).

Substanz C: 2,2-Dibrom-3-oxododecannitril
gelbliches Öl mit $n_D^{20}$ = 1,4956
Analyse
$C_{12}H_{19}ONBr_2$ (MM 353,096)

berechnet (%) : 40,82 C; 5,42 H; 3,97 N; 45,26 Br.
gefunden (%) : 41,2 C; 5,16 H; 3,97 N; 45,4 Br.

Substanz D: 2,2-Dibrom-3-oxotetradecannitril

farblose Kristalle; Fp. 34°C
Analyse
$C_{14}H_{23}ONBr_2$ (MM 381,15)

berechnet (%) : 44,12 C; 6,08 H; 3,67 N; 41,93 Br.
gefunden (%) : 44,2 C; 6,45 H; 3,72 N; 42,6 Br.

Substanz E: 2,2-Dibrom-3-oxoeicosannitril

farblose Kristalle mit Fp. 57°C
Analyse
$C_{20}H_{35}NOBr_2$ (MM 465,32)

berechnet (%) : 51,62 C; 7,58 H; 3,01 N; 34,35 Br.
gefunden (%) : 51,8 C; 7,8 H; 3,0 N; 33.7 Br.

Beispiel 2

In einer Rührapparatur mit Rührer, Tropftrichter, Gaseinleitungsrohr und Rückflußkühler wurden unter Rühren und Einleiten von Stickstoff 28,8 g (0,6 Mol) Natriumhydrid (als 50-gewichtsprozentige Suspension in Weißöl) in 200 ml absolutem Toluol supsendiert und 49,9 g (0,3 Mol) 2-Methoxybenzoesäuremethylester zugegeben. Die Mischung wurde auf 85°C erwärmt. Im Verlauf von 2 Stunden wurden 24,6 g (0,5 Mol) absolutes Acetonitril in die erwärmte Mischung getropft. Danach wurde das Reaktionsgemisch 15 Stunden lang bei 90°C weitergerührt. Das Gemisch wurde dann auf 0°C abgekühlt, unter kräftigem Rühren wurden, vorsichtig 300 ml Eiswasser zugegeben und dann 1 Stunde lang bei 0°C weitergerührt. Anschließend wurde die wäßrige Phase abgetrennt und mit 100 ml Toluol extrahiert. Nach dem Abkühlen auf 0°C wurde die wäßrige Phase mit verdünnter Salzsäure auf pH 1 angesäuert. Das ausgefallene Produkt wurde mit Eiswasser gewaschen und getrocknet. Das Rohprodukt (44 g) wurde aus Isopropanol umkristallisiert; dabei wurden 37,9 g (72% d. Th.) 2-Methoxybenzoylacetonitril mit Fp. 85—88°C erhalten.

8,7 g (0,05 Mol) 2-Methoxybenzoylactonitril wurden in 70 ml Methylenchlorid gelöst. Nach Zugabe einer Lösung von 20 g Kaliumhydrogencarbonat in 100 ml Wasser wurde die Mischung auf −5°C abgekühlt bevor unter kräftigem Rühren innerhalb von 2 Stunden 17,6 g (0,11 Mol) Brom in 30 ml Methylenchlorid zugetropft wurden. Nach beendeter Bromzugabe wurde das Reaktionsgemisch 90 Minuten lang bei −5 bis 0°C weitergerührt. Anschließend wurde überschüssiges Brom durch Zugabe von Hydroxylaminhydrochlorid entfernt, die organische Phase abgetrennt, zweimal mit je 100 ml Wasser gewaschen und über Magneisumsulfat getrocknet. Nach dem Verdampfen des Lösungsmittels im Vakuum verblieben 15,5 g (93% d. Th.) 2-Methoxybenzoyldibromacetonitril (Substanz J) als gelbes Öl mit $n_D^{20}$ = 1,5948.

Analyse
$C_{10}H_7HO_2Br_2$ (MM 333,006)

berechnet (%) : 36,07 C; 2,12 H; 4,21 N; 47,99 Br.
gefunden (%) : 35,90 C; 1,97 H; 4,11 N; 47,80 Br.

$^1H$—NMR-Spektrum (60 MHz; $CDCl_3$)
= 3,92 (s); 6,95—7,77 (m)
IR (Film): 1730 $cm^{-1}$

In analoger Weise wurden die Substanzen F bis I und K bis 0 hergestellt.

Substanz F: Benzoyldibromacetonitril
gelbliches Öl mit $n_D^{20}$ = 1,5982; Sdp. 120°C/0,01 mbar (Dünnschichtverdampfer)
Analyse
$C_9H_5NOBr_2$ (MM 302, 952)

berechnet (%) : 35,68 C; 1,66 H; 4,62 N; 52,75 Br.
gefunden (%) : 34,0 C; 1,5 H; 4,7 N; 53,8 Br.

$^1H$—NMR-Spektrum (60 MHz; $CDCl_3$)
= 8,19 (d,d); 7,m59—7,33 (m)
$^{13}C$—NMR-Spektrum (50MHz; $CDCl_3$)
= 179,9 (s); 135,2 (d); 131,2 (d); 128,8 (d); 128,4 (s); 114,1 (s); 31,4 (s).

Substanz G: 2-Methylbenzoyldibromacetonitril
gelbliches Öl mit $n_D^{20}$ = 1,5949
Analyse
$C_{10}H_7NOBr_2$ (MM 317, 006)

berechnet (%) : 37,89 C; 2,23 H; 4,42 N; N; 50,42 Br.
gefunden (%) : 39,90 C; 1,65 H; 4,46 N; 50,60 Br.

Substanz H: 3-Methylbenzoyldibromacetonitril
gelbliches Öl mit $n_D^{20}$ = 1,5991
Analyse
$C_{10}H_7NOBr_2$ (MM 317, 006)

berechnet (%) : 37,89 C; 2,23 H; 4,42 N; 50,42 Br.
gefunden (%) : 38,20 C; 1,99 H; 4,38 N; 51,5 Br.

Substanz I: 4-Methylbenzoyldibromacetonitril
farblose Kristalle; Fp. 65—69°C
Analyse
$C_{10}H_7NOBr_2$ (MM 317,006)

berechnet (%) : 37,89 C; 2,23 H; 4,42 N; 50,42 Br.
gefunden (%) : 37,40 C; 2,11 H; 4,39 N; 50,40 Br.

Substanz K: 3-Methoxybenzoyldibromacetonitril
gelbliches Öl mit $n_D^{20}$ = 1,6048
Analyse
$C_{10}H_7NOBr_2$ (MM 317,006)

berechnet (%) : 36,07 C; 2,12 H; 4,21 N; 47,99 Br.
gefunden (%) : 36,00 C; 2,07 H; 4,26 N; 48,8 Br.

Substanz L: 4-Methoxybenzoylbromacetonitril
farblose Kristalle; Fp 57—62°C
Analyse
$C_{10}H_7NO_2Br_2$ (MM 33,006)

berechnet (%) : 36,07 C; 2,12 H; 4,21 N; 47,99 Br.
gefunden (%) : 35,80 C; 2,09 H; 4,26 N; 47,60 Br.

Substanz M: 4-Brombenzoyldibromacetonitril
farblose Kristalle; Fp. 38—44°C
Analyse
$C_9H_4NOBr_3$ (MM 381, 887)

berechnet (%) : 28,31 C; 1,06 H; 3,67 N; 62,78 Br.
gefunden (%) : 27,90 C; 0,96 H; 3,66 N; 61,90 Br.

Substanz N: 1-Naphthoyldibromacetonitril
farblose Kristalle; Fp. 48—51°C Analyse
$C_{13}H_7NOBr_2$ (MM 353,036)

berechnet (%) : 44,23 C; 2,00 H; 3,97 N; 45,27 Br.
gefunden (%) : 43,20 C; 1,91 H; 3,80 N; 44,8 Br.

Substanz O: 2-Naphthoyldibromacetonitril
farblose Kristalle: Fp 61—67°C
Analyse
$C_{13}H_7NOBr_2$ (MM 353,036)

berechnet (%) : 44,23 C; 2,00 H; 3,97 N; 45,27 Br.
gefunden (%) : 43,50 C; 1,91 H; 3,75 N; 45,00 Br.

Die gemäß den Beispielen 1 und 2 dargestellten 2,2-Dibrom-3-oxonitrile sind in der nachstehenden Tabelle I zusammen mit ihren physikalischen Daten zusammengefaßt.

TABELLE I

2,2-Dibrom-3-oxonitrile der Formel R—CO—$CBr_2$—CN

| Substanz | R | Sdp. (°C/mbar) | Fp (°C) | $n_D^{20}$ |
|---|---|---|---|---|
| A | n-$C_5H_{11}$ | 95/0,01 | Öl | 1,5055 |
| B | n-$C_7H_{15}$ | 120,0,01 | Öl | 1,4978 |
| C | n-$C_9H_{19}$ | | Öl | 1,4956 |
| D | n-$C_{11}H_{23}$ | — | 34 | |
| E | n-$C_{17}H_{35}$ | — | 57 | — |
| F | 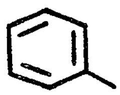 | 120/0,01 | — | 1,5982 |
| G | 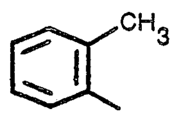 | | Öl | 1,5949 |
| H | 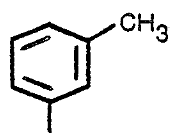 | — | Öl | 1,5991 |
| I | 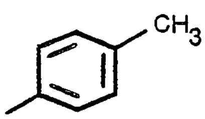 | — | 65—69 | — |
| J | 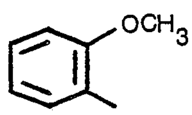 | — | Öl | 1,5948 |
| K | 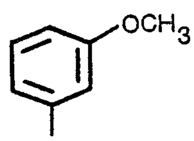 | — | Öl | 1,6048 |
| L | 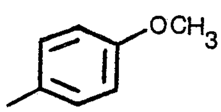 | — | 57—62 | — |
| M | 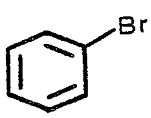 | — | 38—44 | — |
| N | 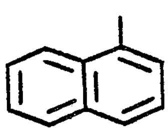 | — | 48—51 | — |
| O | 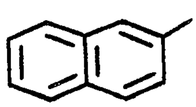 | — | 61—67 | — |

B. Antimikrobielle Wirksamkeit

I. Mikrobistatische Wirksamkeit

Die mikrobistatische Wirksamkeit der erfindungsgemäßen Substanzen A bis O wurde gegenüber folgenden Testkeimsuspensionen bestimmt.

| | |
|---|---|
| 1. Staphylococcus aureus | $2 \times 10^9$ Keime/ml |
| 2. Escherichia coli | $2 \times 10^9$ Keime/ml |
| 3. Pseudomonas aeruginosa | $5 \times 10^8$ Keime/ml |
| 4. Candida albicans | $2 \times 10^8$ Keime/ml |
| 5. Aspergillus niger | $5 \times 10^7$ Keime/ml |
| 6. Penicillium camerunense | $5 \times 10^7$ Keime/ml |

Die Hemmkonzentrationen der zu untersuchenden Verbindungen wurden mit Hilfe des Verdünnungstestes nach den Richtlinien für die Prüfung chemischer Desinfektionsmittel der Deutschen Gesellschaft für Hygiene und Mikrobiologie (1972) ermittelt. Die Versuche wurden in sterilen Reagenzröhrchen ausgeführt, die für die Keime 1 bis 3 Standard-I-Bouillon (pH 7,5 Merck) und für die Keime 4 bis 6 Würzebouillon (pH 5,5, Merck) enthielten. Nach der Zugabe der Wirkstoffe betrug das Nährlösungsvolumen in den Röhrchen jeweils 5 ml. Anschließend wurden jeweils 0,1 ml der Testkeimsuspension der angegebenen Konzentration in die Röhrchen gebracht. Die mit Bakterien beimpften Nährlösungsproben wurden 3 Tage lang bei 37°C im Brutschrank aufbewahrt. Die mit Pilzen beimpften Proben wurden 4 Tage lang bei 30°C bebrütet. Danach wurde festgestellt, welche dem Nährmedium zugeführte Wirkstoffkonzentration des Wachstum der Keime gerade noch gehemmt hatte. Der auf diese Weise gefundene Wert wurde als Hemmkonzentration bezeichnet. Folgende Wirkstoffkonzentrationen in ppm wurden getestet: 1 000, 500, 250, 100, 50 und 10.

Für die Substanzen A bis O wurden die in der nachstehenden Tabelle II wiedergegebenen Hemmkonzentrationen ermittelt.

TABELLE II

Hemmkonzentrationen (in ppm) der Substanzen A bis O im Verdünnungstest

| Substanz | Testkeim | | | | | |
|---|---|---|---|---|---|---|
| | 1 | 2 | 3 | 4 | 5 | 6 |
| A | 50 | 100 | 100 | 50 | 50 | 50 |
| B | 50 | 100 | 100 | 50 | 50 | ≤10 |
| C | 50 | 100 | 100 | 50 | 50 | 50 |
| D | 50 | 100 | 100 | 50 | 50 | 50 |
| E | | | | | | |
| F | 100 | 50 | 50 | 50 | ≤10 | ≤10 |
| G | 100 | 250 | 500 | 100 | 50 | 50 |
| H | 100 | 100 | 100 | 50 | ≤10 | ≤10 |
| I | 100 | 100 | 100 | 50 | 50 | 50 |
| J | 100 | 250 | 100 | 50 | 50 | 50 |
| K | 100 | 100 | 100 | 50 | 50 | 50 |
| L | 100 | 100 | 100 | 50 | 50 | 50 |
| M | 100 | 100 | 100 | 50 | 50 | 50 |
| N | 250 | 500 | 500 | 100 | 50 | 50 |
| O | 100 | 100 | 100 | 100 | ≤10 | ≤10 |

2. Mikrobizide Wirksamkeit

Die mikrobizide Wirksamkeit der erfindungsgemäßen Substanzen A bis O wurde gegenüber folgenden Testkeimsuspensionen bestimmt:

| | |
|---|---|
| 1. Staphylococcus aureus | $2 \times 10^9$ Keime/ml |
| 2. Escherichia coli | $2 \times 10^9$ Keime/ml |
| 3. Pseudomonas aeruginosa | $5 \times 10^8$ Keime/ml |
| 4. Candida albicans | $2 \times 10^8$ Keime/ml |
| 5. Aspergillus niger | $5 \times 10^7$ Keime/ml |
| 6. Penicillium camerunense | $5 \times 10^7$ Keime/ml |

Die Abtötungszeiten der zu untersuchenden Produkte wurden mit Hilfe des Suspensionstests ermittelt. Unter Verwendung von Wasser einer Härte von 17° dH wurden Testlösungen hergestellt, die 2000 bzw. 500 ppm Wirkstoff enthielten. Bei Raumtemperatur wurden jeweils 0,1 ml Testkeimsuspension in Reagenzgläser pipettiert und mit jeweils 10 ml der oben beschriebenen Testlösungen vermischt. Nach unterschiedlichen Einwirkungszeiten bis zu 60 Minuten wurden den Reagenzgläsern mit Hilfe einer Impföse ca. 0,05 ml Material entnommen und auf einem Nähragar, der als Enthemmer 3% Tween 80 und 0,3% Lecithin enthielt, ausgestrichen. Das Nährmedium bestand für die Keime 1 bis 3 aus 2,5 gewichtsprozentiger Standard-I-Bouillon (Merck) und für die Keime 4 bis 6 aus Würzebouillon pH 5 (Merck), die zur Verfestigung jeweils 1,2 Gewichtsprozent Agar enthielten. Die Proben wurden bei 37°C bzw. 30°C bebrütet. Nach 2 bis 3 Tagen wurden die Kulturen makroskopisch auf Wachstum beurteilt und auf diesem Weg die Abtötungszeit oder der Restkeimgehalt ermittelt. Die dabei gefundenen Ergebnisse sind in der nachfolgenden Tabelle II wiedergegeben.

In der Tabelle III bedeuten "+" weniger als 50, "++" weniger als 200 und "+++" mehr als 200 Restkeime nach 60 Minuten Einwirkungszeit. Die in den einzelnen Spalten angegebenen Zahlenwerte stehen für die Einwirkungszeit in Minuten.

TABELLE III

Abtötungszeiten der Substanzen A bis O im Suspensionstest

| Substanz | Konz. (ppm) | Testkeim 1 | 2 | 3 | 4 | 5 | 6 |
|---|---|---|---|---|---|---|---|
| A | 2000 | ≤15 | ≤15 | ≤15 | ≤15 | ≤15 | ≤15 |
| | 500 | ≤15 | 60 | 60 | ≤15 | ≤15 | 60 |
| B | 2000 | ≤15 | ≤15 | ≤15 | ≤15 | ≤15 | ≤15 |
| | 500 | ≤15 | 60 | 60 | 60 | ≤15 | 60 |
| C | 2000 | ≤15 | ≤15 | ≤15 | ≤15 | ≤15 | ≤15 |
| | 500 | ≤15 | 60 | ≤15 | ≤15 | ≤15 | 60 |
| D | 2000 | ≤15 | +++ | 60 | 60 | 60 | +++ |
| | 500 | 60 | +++ | +++ | +++ | +++ | +++ |
| E | | | | | | | |
| F | 2000 | ≤15 | ≤15 | ≤15 | ≤15 | ≤15 | ≤15 |
| | 500 | + | 60 | ≤15 | 60 | 60 | 60 |
| G | 2000 | 60 | +++ | 60 | 60 | ++ | +++ |
| | 500 | + | +++ | +++ | +++ | +++ | +++ |
| H | 2000 | 60 | 60 | ≤15 | ≤15 | 60 | ≤15 |
| | 500 | ++ | ++ | 60 | ++ | +++ | +++ |
| I | 2000 | 60 | 60 | ≤15 | 60 | 60 | ++ |
| | 500 | + | ++ | 60 | ++ | ++ | +++ |

TABELLE III (Fortsetzung)

| Substanz | Konz. (ppm) | Testkeim | | | | | |
|---|---|---|---|---|---|---|---|
| | | 1 | 2 | 3 | 4 | 5 | 6 |
| J | 2000 | 60 | 60 | ≤15 | ≤15 | 60 | ≤15 |
| | 500 | +++ | +++ | + | +++ | +++ | +++ |
| K | 2000 | ++ | 60 | ≤15 | 60 | + | ≤15 |
| | 500 | +++ | +++ | +++ | +++ | +++ | +++ |
| L | 2000 | +++ | 60 | ≤15 | 60 | 60 | +++ |
| | 500 | +++ | + | 60 | ++ | ++ | +++ |
| M | 2000 | 60 | 60 | ≤15 | ≤15 | 60 | +++ |
| | 500 | +++ | ++ | 60 | 60 | +++ | +++ |
| N | 2000 | ≤15 | 60 | 60 | 60 | ++ | +++ |
| | 500 | 60 | 60 | 60 | 60 | ++ | +++ |
| O | 2000 | ≤15 | ≤15 | ≤15 | ≤15 | ≤15 | ++ |
| | 500 | ≤15 | ≤15 | ≤15 | ≤15 | 60 | +++ |

**Patentanspruch**

Verfahren zur Herstellung von 2,2-Dibrom-3-oxonitrilen der Formel I,

$$R—CO—CBr_2—CN \quad (I)$$

in der R einen geradkettigen oder verzweigten Alkylrest mit 3 bis 21 Kohlenstoffatomen, einen gegebenenfalls mit Alkylresten substituierten cycloaliphatischen Rest mit 3 bis 6 Ringkohlenwasserstoffatomen, oder einen gegebenenfalls mit Alkylresten, Alkoxyresten, Fluor- oder Bromatomen oder Nitrogruppen substituierten Phenyl- oder Naphtylrest darstellt, bei dem man einen Carbonsäureester der Formel II,

$$R—CO—OR' \quad (II)$$

in der R die für die Formel I angegebene Bedeutung hat und in der R' einen geradkettigen oder verzweigten Alkylrest mit 1 bis 4 Kohlenstoffatomen darstellt, in Gegenwart einer starken Base umsetzt, und das entstandene 3-Oxonitril mit Brom umsetzt, dadurch gekennzeichnet, daß man den Carbonsäureester der Formel II in Gegenwart von Natriummethylat mit im Überschuß vorhandenem Acetonitril umsetzt und den bei der Reaktion entstehenden Alkohol zusammen mit Acetonitril laufend aus dem Reaktionsgemisch abdestilliert, das entstandene 3-Oxonitril aus dem Reaktionsgemisch isoliert und in einem gegen Brom indifferenten Lösungsmittel und in Gegenwart eines Bromwasserstoffacceptors, gegebenenfalls unter Kühlung mit Brom umsetzt und das entstandene 2,2-Dibromoxonitril der Formel I aus dem Reaktionsgemisch isoliert.

**Revendication**

Procédé de préparation de 2,2-dibromo 3-oxo nitriles de formule I

$$R—CO—CBr_2—CN \quad (I)$$

dans laquelle R représente un radical alcoyle linéaire ou ramifié ayant de 3 à 21 atomes de carbone, un radical cycloaliphatique, dont le cadre comporte de 3 à 6 atomes de carbone éventuellement substitué avec des radicaux alcoyle ou un radical phényle ou naphtyle éventuellement substitués avec des radicaux alcoyle, des radicaux alcoxy, des atomes de fluor ou de brome, ou des groupes nitro, par lequel on fait réagir en présence d'une base forte un ester d'acide carboxylique de formule II.

$$R—CO—OR' \quad II$$

dans laquelle R a la même signification que dans la formule I et R' représente un radical alcoyle linéaire ou

ramifié ayant de 1 à 4 atomes de carbone et on fait réagir le 3 — oxo nitrile formé avec du brome, procédé caractérisé en ce que l'on fait réagir l'ester carboxylique de formule II avec un excès d'acétronitrile, en présence de méthylate de sodium, e l'on sépare du mélange réactionnel, par distillation, l'alcool qui s'est formé en cours de réaction, avec l'acétronitrile, l'on isole du mélange réactionnel le 3 - oxo nitrile formé et on le fait réagir avec du brome dans un solvant inerte vis-à-vis de ce dernier et en présence d'un accepteur d'acide bromhydrique, le cas échéant sous refroidissement et l'on isole du mélange réactionnel le 2,2 — dibromo oxonitrile de formule I formé.

**Claim**

A process for the production of 2,2-dibromo-3-oxonitriles corresponding to formula I

$$R—CO—CBr_2—CN \qquad (I)$$

in which R is a linear or branched $C_{3-21}$ alkyl radical, an optionally alkyl-substituted cycloaliphatic radical containing 3 to 6 ring carbon atoms or an optionally alkyl-, alkoxy-, fluorine- or bromine- or nitro-substituted phenyl or naphthyl radical, in which a carboxylic acid ester corresponding to formula II

$$R—CO—OR' \qquad (II)$$

in which R is as defined for formula I and R' is a linear or branched $C_{1-4}$ alkyl radical, is reacted in the presence of a strong base and the 3-oxonitrile formed is reacted with bromine, characterized in that the carboxylic acid ester corresponding to formula II is reacted with acetonitrile present in excess in the presence of sodium methylate and the alcohol formed during the reaction is continuously distilled off from the reaction mixture together with acetonitrile, the 3-oxonitrile formed is isolated from the reaction mixture and reacted with bromine, optionally with cooling, in a solvent inert to bromine and in the presence of a hydrogen bromide acceptor and the 2,2-dibromoxonitrile of formula I formed is isolated from the reaction mixture.